(19) Europäisches Patentamt
European Patent Office
Office européen des brevets

(11) **EP 3 180 088 B1**

(12) **EUROPÄISCHE PATENTSCHRIFT**

(45) Veröffentlichungstag und Bekanntmachung des Hinweises auf die Patenterteilung:
**16.10.2019 Patentblatt 2019/42**

(21) Anmeldenummer: **15731073.1**

(22) Anmeldetag: **25.06.2015**

(51) Int Cl.:
***A61K 8/44*** (2006.01) ***A61K 8/41*** (2006.01)
***A61Q 5/06*** (2006.01) ***A61Q 5/10*** (2006.01)
***A61Q 5/12*** (2006.01) ***A61K 8/893*** (2006.01)
***A61K 8/894*** (2006.01)

(86) Internationale Anmeldenummer:
**PCT/EP2015/064352**

(87) Internationale Veröffentlichungsnummer:
**WO 2016/023666 (18.02.2016 Gazette 2016/07)**

(54) **OXIDATIONSFÄRBEMITTEL MIT SPEZIELLEN NICHTIONISCHEN SILICONPOLYMEREN**

OXIDATION DYEING AGENT HAVING SPECIFIC NON-IONIC SILICONE POLYMERS

COMPOSITION DE COLORATION PAR OXYDATION AVEC DES POLYMÈRES DE SILICONE NON-IONIQUES PARTICULIERS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.08.2014 DE 102014216202**

(43) Veröffentlichungstag der Anmeldung:
**21.06.2017 Patentblatt 2017/25**

(73) Patentinhaber: **Henkel AG & Co. KGaA**
**40589 Düsseldorf (DE)**

(72) Erfinder:
- **KERL, Sylvia**
**22763 Hamburg (DE)**
- **BIETZ, Susanne**
**25336 Elmshorn (DE)**

(56) Entgegenhaltungen:
**EP-A1- 0 547 790**
**EP-A1- 2 329 809**
**EP-A2- 2 556 816**
**EP-A2- 2 559 456**
**US-A1- 2002 144 356**
**US-A1- 2009 081 146**
**US-A1- 2010 017 971**
**US-A1- 2010 229 314**
**US-A1- 2013 125 317**
**US-A1- 2013 156 716**
**US-A1- 2014 190 999**

- **DATABASE GNPD [Online] MINTEL; 1. Juli 2014 (2014-07-01), "Anti-Grey Color Gel", XP002742641, Database accession no. 2574083**
- **DATABASE GNPD [Online] MINTEL; 1. Januar 2009 (2009-01-01), "Ammonia Free Permanet Colouring Cream", XP002742642, Database accession no. 1044299**
- **DATABASE GNPD [Online] MINTEL; 1. Januar 2013 (2013-01-01), "Hair Colorant", XP002742643, Database accession no. 1984658**
- **DATABASE GNPD [Online] MINTEL; 1. September 2009 (2009-09-01), "Loss Fortifying Hair Colour", XP002742644, Database accession no. 1176630**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 180 088 B1

Printed by Jouve, 75001 PARIS (FR)

## Beschreibung

Die vorliegende Erfindung betrifft kosmetische Mittel zur Färbung keratinischer Fasern, welche spezielle nichtionische Siliconpolymere enthalten.

Weiterhin betrifft die vorliegende Erfindung eine Verpackungseinheit (Kit-of-parts), enthaltend ein erfindungsgemäßes kosmetisches Mittel sowie eine Oxidationsmittelzubereitung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine bedeutende Rolle. Abgesehen von Blondiermitteln, welche eine oxidative Aufhellung der Haare durch Abbau der natürlichen Haarfarbstoffe bewirken, ist im Bereich der Haarfarbveränderung die oxidative Haarfärbung von wesentlicher Bedeutung.

Für dauerhafte, intensive Färbungen mit entsprechenden Echtheitseigenschaften werden so genannte Oxidationsfärbemittel verwendet. Solche Färbemittel enthalten üblicherweise Oxidationsfarbstoffvorprodukte, auch als Entwicklerkomponenten und Kupplerkomponenten bezeichnet. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Für natürlich wirkende Färbungen muss üblicherweise eine Mischung aus einer größeren Zahl von Oxidationsfarbstoffvorprodukten (nachfolgend als OFV bezeichnet) eingesetzt werden; in vielen Fällen werden weiterhin direktziehende Farbstoffe (nachfolgend als DZ bezeichnet) zur Nuancierung verwendet.

Zumeist weisen oxidative Färbemittel zur Stabilisierung der Farbstoffvorprodukte während der Lagerung und zur Reaktionsbeschleunigung während der oxidativen Anwendung einen alkalischen pH-Wert auf, welcher mit Alkalisierungsmitteln, wie Alkanolaminen, Ammoniak oder anorganischen Basen, eingestellt wird.

Die vorgenannten Oxidationsfarbstoffvorprodukte (OFV) und Alkalisierungsmittel sind üblicherweise in einen kosmetisch geeigneten Träger, beispielsweise einer Creme oder einem Gel, eingearbeitet. Der Träger gewährleistet eine homogene Verteilung und eine ausreichende Verweilzeit des oxidativen Färbemittels auf dem Haar.

Kommerzielle Oxidationsfärbemittel werden üblicherweise in Produktserien formuliert, welche einen standardisierten Träger umfassen, der mit der Nuancen-spezifischen OFV-Kombination und Alkalisierungsmitteln möglichst uneingeschränkt kombiniert werden kann.

Verbraucher können die mit einem Haarfärbemittel erzielbare Färbung der Haare in der Regel einem Hinweis auf der Verpackung des Haarfärbemittels und/oder einer der Verpackung beiliegenden Farbkarte entnehmen. Für den Verbraucher ist es dabei sehr wichtig, dass das Ergebnis der Färbung möglichst genau mit der vom Hersteller angegebenen Farbe übereinstimmt.

Haarfärbemittel werden daher vor der Markteinführung umfassend und aufwändig auf die erzielbare Farbe sowie auf eine Vielzahl von Anwendungseigenschaften hin getestet. Jedoch berücksichtigen diese Tests die Wechselwirkungen zwischen OFV sowie gegebenenfalls DZ und dem standardisierten Träger immer nur für einen bestimmten standardisierten Träger. Seitens der Hersteller besteht regelmäßig der Wunsch, eine Haarfärbemittelserie gezielt auf die speziellen Bedürfnisse bestimmter Verbrauchergruppen abzustimmen, indem dem standardisierten Träger entsprechende Wirk- oder Pflegestoffe zugesetzt werden. Für Verbraucher mit stärker geschädigtem Haar wäre beispielsweise der Zusatz von einem oder mehreren Pflegewirkstoffen mit reparierender Wirkung empfehlenswert; für Verbraucher mit feinem Haar wäre der Zusatz von einem oder mehreren die Haarstruktur stärkenden Wirkstoffen empfehlenswert.

Das Ergebnis der Färbung hängt jedoch nicht allein von der eingesetzten Kombination der OFV und gegebenenfalls DZ ab, sondern wird insbesondere auch von den Inhaltsstoffen des Trägers beeinflusst. Beispielsweise kann der Zusatz von Pflege- und/oder Wirkstoffen zu den standardisierten Trägern zu einer Veränderung des Aufziehvermögens der unter dem Einfluss des Oxidationsmittels gebildeten bzw. der direkt eingesetzten Farbstoffe auf die keratinische Fasern und somit zu einem gegenüber dem standardisierten Träger stark veränderten Färbeergebnis führen.

Derartige Farbunterschiede bzw. veränderte Färbeergebnisse werden im Sinne der vorliegenden Anmeldung als "Farbverschiebung" bezeichnet. Diese Farbverschiebung, auch als dE oder $\Delta$E bezeichnet, lässt sich farbmetrisch mit einem Farbmessgerät bestimmen, mit welchem die Farben im L*,a*,b*-Farbraum vermessen werden, beispielsweise mit einem Farbmessgerät der Firma Datacolor, Typ Spectraflash 450.

Unter dem L*,a*,b*-Farbraum wird der CIELAB-Farbenraum verstanden. Der L-Wert steht hierbei für die Helligkeit der Färbung (schwarz-weiß-Achse); je größer der Wert für L ist, desto heller ist die Färbung. Der a-Wert steht für die rot-grün-Achse des Systems; je größer dieser Wert ist, desto mehr ist die Färbung ins Rote verschoben. Der b-Wert steht für die gelb-blau-Achse des Systems; je größer dieser Wert ist, desto mehr ist die Färbung ins Gelbe verschoben.

Die Farbverschiebung $\Delta$E, also die Farbdifferenz zwischen zwei (Haar-)Farben, für welche jeweils eine L*,a*,b*-Wertekombination bestimmt wurde, wird gemäß folgender Formel berechnet:

$$\Delta E = ((L_i - L_0)^2 + (a_i - a_0)^2 + (b_i - b_0)^2)^{1/2}$$

ao, bo und Lo sind die L*, a* und b*-Werte der unter Verwendung des standardisierten Trägers ausgefärbten Haarsträhnen, während $a_i$, $b_i$ und $L_i$ die L*, a* und b*-Werte sind, welche für Ausfärbungen unter Verwendung von Pflege- und/oder Wirkstoffen in dem standardisierten Träger erhalten werden. Je größer der Wert für $\Delta E$ ist, desto stärker ausgeprägt ist die Farbdifferenz oder "Farbverschiebung". Farbdifferenzen mit $\Delta E < 1$ sind für das menschliche Auge nicht wahrnehmbar. Farbdifferenzen mit $\Delta E < 2$ sind lediglich für das geschulte Auge sichtbar. Farbdifferenzen mit $\Delta E > 2$ sind auch für das ungeschulte Auge sichtbar.

Im ungünstigsten Fall bewirkt die Zugabe eines Zusatzstoffs zu einem standardisierten Träger eine Farbverschiebung gegenüber dem Standardträger ohne Zusatzstoffe von $\Delta E > 2$, welche daher auch für das ungeschulte Auge des Verbrauchers sichtbar ist. Um zu vermeiden, dass bei jeder Zugabe von Zusatzstoffen zu standardisierten Trägern aufwändige Tests hinsichtlich der erzielbaren Haarfärbung und gegebenenfalls der Echtheitseigenschaften durchgeführt werden müssen, ist es daher wünschenswert, Wirk- und Pflegestoffe für das Haar zu identifizieren, deren Zugabe keine oder zumindest nur eine geringe Farbverschiebung bewirkt.

Aus EP 547790A1, US 2002/0144356A1 und EP 2559456A1 sind Färbemittel für keratinische Fasern bekannt, welche mindestens ein Oxidationsfarbstoffvorprodukt (OFV) und/oder mindestens einen direktziehenden Farbstoff (DZ) sowie mindestens ein nichtionisches Siliconpolymer mit einer höheren Anzahl von EO-Gruppen enthalten.

In US 2010/0229314 werden ebenfalls Haarfärbemittel beschrieben, welche mindestens ein Oxidationsfarbstoffvorprodukt (OFV) und/oder mindestens einen direktziehenden Farbstoff (DZ) sowie mindestens ein nichtionisches Siliconpolymer enthalten.

In allen Dokumenten werden die nichtionischen Silikonpolymere in höheren Mengen eingesetzt, um die Farbtiefe von Haarfärbungen zu verbessern.

Der vorliegenden Anmeldung lag die Aufgabe zu Grunde, kosmetische Mittel zur Farbveränderung keratinischer Fasern bereitzustellen, welche ein oder mehrere ausgewählte Pflege- und Wirkstoffe enthalten, die keine oder nur eine minimale Farbverschiebung hervorrufen.

Es wurde nun überraschenderweise gefunden, dass der Zusatz von mindestens einem speziellen nichtionischen Siliconpolymer in kosmetischen Mitteln zur Färbung keratinischer Fasern, insbesondere menschlicher Haare, zu einer verbesserten Pflege, insbesondere zu einer verbesserten Kämmbarkeit, bei gleichzeitig minimaler Farbverschiebung von $\Delta E < 2$ führt.

Ein erster Gegenstand der Erfindung ist daher ein kosmetisches Mittel zur Farbveränderung keratinischer Fasern, umfassend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,

b) mindestens ein nichtionisches Siliconpolymer in einer Gesamtmenge von 0,005 bis 0,5 Gew.-%, bezogen auf das Gesamtgewicht des kosmetischen Mittels, enthaltend mindestens eine Struktureinheit der Formel (I) und mindestens eine Struktureinheit der Formel (II)

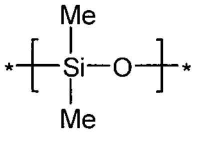
(I)

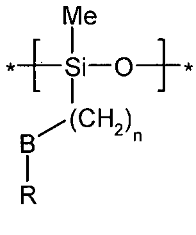
(II),

worin

n für ganze Zahlen von 1 bis 5 steht,

B für eine direkte Bindung oder ein Heteroatom aus der Gruppe von O, N oder S steht, und

R für eine Gruppe *-$(CH_2CH_2O)_x$-H oder *-$(CH_2CHMeO)_y$-H oder *-$(CH_2CH_2O)_x(CH_2CHMeO)_y$-H steht, worin x und y, jeweils unabhängig voneinander, für ganze Zahlen von 2 bis 20 stehen,

wobei das kosmetische Mittel mindestens ein nichtionisches Siliconpolymer der Formel (III) enthält

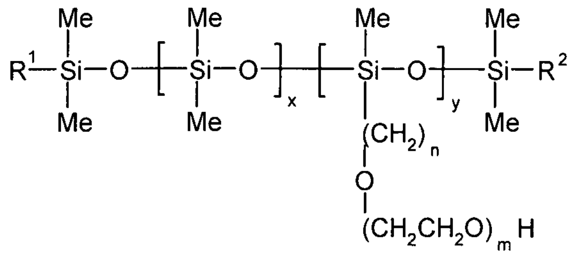

(III),

worin

$R^1$ und $R^2$, jeweils unabhängig voneinander, für eine Methylgruppe oder eine Hydroxylgruppe stehen,
x und y, jeweils unabhängig voneinander, für ganze Zahlen von 1 bis 1.000 stehen,
n für die ganzen Zahlen 2 oder 3 steht, und
m für ganze Zahlen von 5 bis 9 steht.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol "*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Unter dem Begriff "keratinische Fasern / Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Färbung von menschlichen Haaren verwendet werden.

Weiterhin werden unter dem Begriff "nichtionische Siliconpolymere" im Rahmen der vorliegenden Erfindung Siliconpolymere verstanden, welche keine permanent anionischen oder permanent kationischen Gruppen sowie keine anionisierbaren oder kationisierbaren Gruppen, wie beispielsweise Carbonsäuregruppen oder Amingruppen, aufweisen.

Zudem wird unter dem Begriff "Kämmbarkeit" im Rahmen der vorliegenden Erfindung sowohl die Kämmbarkeit der nassen Faser, als auch die Kämmbarkeit der trockenen Faser verstanden.

Darüber hinaus werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen Mittels bezieht sich vorliegend - sofern nichts anderes angegeben ist - auf die Gesamtmenge an Aktivsubstanz der jeweiligen Komponente. Weiterhin bezieht sich die Angabe der Gesamtmenge in Bezug auf die Komponenten des kosmetischen Mittels - sofern nichts anderes angegeben ist - auf das Gesamtgewicht des oxidationsmittelfreien erfindungsgemäßen kosmetischen Mittels.

Die erfindungsgemäßen Mittel enthalten einen kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger enthält im Sinne der Erfindung enthält mindestens 30 Gew.-%, insbesondere mindestens 50 Gew.-%, Wasser, bezogen auf das Gesamtgewicht des kosmetischen Mittels.

Unter wässrig-alkoholischen Trägern sind im Sinne der vorliegenden Erfindung wasserhaltige Zusammensetzungen, enthaltend einen $C_1$-$C_4$-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Das erfindungsgemäße kosmetische Mittel enthält als ersten wesentlichen Bestandteil a) eine Verbindung,

ausgewählt aus der Gruppe von Oxidationsfarbstoffvorprodukten (OFV), direktziehenden Farbstoffe (DZ) sowie deren Mischungen.

In einer bevorzugten Ausführungsform enthalten erfindungsgemäße Mittel mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige Verbindungen im erfindungsgemäßen kosmetischen Mittel enthalten sein. In einer bevorzugten Ausführungsform enthalten die erfindungsgemäßen kosmetischen Mittel daher mindestens ein Oxidationsfarbstoffvorprodukt vom Entwicklertyp. Es kann im Rahmen der vorliegenden Erfindung jedoch auch vorgesehen sein, dass die erfindungsgemäßen kosmetischen Mittel mindestens ein Oxidationsfarbstoffvorprodukt vom Kupplertyp enthalten. Besonders gute Ergebnisse in Bezug auf die Färbung keratinischer Fasern werden erhalten, wenn die erfindungsgemäßen kosmetischen Mittel mindestens ein Oxidationsfarbstoffvorprodukt vom Entwicklertyp und mindestens ein Oxidationsfarbstoffvorprodukt vom Kupplertyp enthalten.

Die Entwickler- und Kupplerkomponenten werden üblicherweise in freier Form eingesetzt. Bei Substanzen mit Aminogruppen kann es jedoch bevorzugt sein, deren Salzform, insbesondere in Form der Hydrochloride und Hydrobromide oder der Sulfate, einzusetzen.

Erfindungsgemäß sind kosmetische Mittel bevorzugt, welche die Entwickler- und/oder Kupplerkomponenten jeweils in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthalten.

In einer weiteren bevorzugten Ausführungsform ist das erfindungsgemäße kosmetische Mittel daher dadurch gekennzeichnet, dass es ein Oxidationsfarbstoffvorprodukt vom Entwickler und/oder Kupplertyp in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthält.

Geeignete Oxidationsfarbstoffvorprodukte vom Entwicklertyp sind beispielsweise p-Phenylendiamin und dessen Derivate. Bevorzugte p-Phenylendiamine werden ausgewählt aus einer oder mehrerer Verbindungen der Gruppe, welche gebildet wird aus p-Phenylendiamin, p-Toluylendiamin, 2-Chlorp-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, welche mindestens zwei aromatische Kerne enthalten, die mit Amino- und/oder Hydroxylgruppen substituiert sind. Bevorzugte zweikernige Entwicklerkomponenten werden ausgewählt aus N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan, Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol, 4-Amino-2-(diethylaminomethyl)phenol sowie deren physiologisch verträglichen Salze.

Ferner kann die Entwicklerkomponente ausgewählt sein aus o-Aminophenol und dessen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind 2,4,5,6-Tetraaminopyrimidin, 4-Hydroxy-2,5,6-triaminopyrimidin und deren physiologisch verträgliche Salze. Ein bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, welche gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxy-ethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen.

Besonders bevorzugte Entwicklerkomponenten sind p-Toluylendiamin, 2-(2-Hydroxyethyl)-p-phenylen-diamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Gemäß einer weiteren bevorzugten Ausführungsform der vorliegenden Erfindung enthält das erfindungsgemäße kosmetische Mittel als Oxidationsfarbstoffvorprodukt neben mindestens einer Entwicklerkomponente weiterhin zusätzlich mindestens eine Kupplerkomponente. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus

(A) m-Aminophenol und dessen Derivaten, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivaten, wie 2-Amino-5-ethylphenol,
(C) m-Diaminobenzol und dessen Derivaten, wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,
(D) o-Diaminobenzol und dessen Derivaten,
(E) Di- beziehungsweise Trihydroxybenzolderivaten, insbesondere Resorcin, 2-Chlorresorcin, 4-Chlorresorcin, 2-Methylresorcin und 1,2,4-Trihydroxybenzol,
(F) Pyridinderivaten, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxy-pyridin,
(G) Naphthalinderivaten, wie 1-Naphthol und 2-Methyl-1-naphthol,
(H) Morpholinderivaten, wie 6-Hydroxybenzomorpholin,
(I) Chinoxalinderivaten,
(J) Pyrazolderivaten, wie 1-Phenyl-3-methylpyrazol-5-on,
(K) Indolderivaten, wie 6-Hydroxyindol,
(L) Pyrimidinderivaten oder
(M) Methylendioxybenzolderivaten, wie 1-(2'-Hydroxyethyl)-amino-3,4-methylendioxybenzol sowie deren physiologisch verträglichen Salze.

Erfindungsgemäß bevorzugte Kupplerkomponenten sind ausgewählt aus der Gruppe, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie deren physiologisch verträgliche Salze.

In einer besonders bevorzugten Ausführungsform der vorliegenden Erfindung sind die erfindungsgemäßen kosmetischen Mittel dadurch gekennzeichnet, dass sie als Oxidationsfarbstoffvorprodukt mindestens eine Entwicklerkomponente, ausgewählt aus der Gruppe von p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol, deren physiologisch verträglichen Salze sowie deren Mischungen, und mindestens eine Kupplerkomponente, ausgewählt aus der Gruppe von Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin, deren physiologisch verträglichen Salze sowie deren Mischungen, enthalten.

Um eine ausgewogene und subtile Nuancenausbildung zu erhalten, kann es im Rahmen der vorliegenden Erfindung auch vorgesehen sein, dass die erfindungsgemäßen kosmetischen Mittel zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, welche mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das erfindungsgemäße kosmetische Mittel die direktziehenden Farbstoffe in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels.

Als zweiten wesentlichen Bestandteil b) enthalten die erfindungsgemäßen kosmetischen Mittel mindestens ein spezielles nichtionisches Siliconpolymer. Der Zusatz dieses Siliconpolymers führt zu einer verbesserten Pflege, insbesondere Nasskämmbarkeit, ohne jedoch eine für das ungeschulte menschliche Auge sichtbare Farbverschiebung zu verursachen, d. h. die durch den Zusatz spezieller nichtionischer Siliconpolymere verursachte Farbveränderung $\Delta E$ ist kleiner als 2.

Der Einsatz dieser speziellen nichtionischen Siliconpolymere resultiert in einer erhöhten Pflege der keratinischen Fasern nach der Farbveränderung und führt gleichzeitig nicht zu einer sichtbaren Farbverschiebung des Färbeergebnisses für das ungeschulte menschliche Auge. Das zuvor genannte Siliconpolymer kann daher in beliebigen standardisierten Trägern eingesetzt werden, ohne dass eine Anpassung der Oxidationsfarbstoffvorprodukte und gegebenenfalls der Direktzieher zum Ausgleich der durch den Zusatz verursachten Farbverschiebung erforderlich ist.

Bevorzugt weist das mindestens eine nichtionische Siliconpolymer b) ein mittleres Molekulargewicht $M_w$ von 350 bis 200.000 Da, vorzugsweise von 500 bis 180.000 Da, bevorzugt von 1.000 bis 150.000 Da, insbesondere von 3.000 bis 100.000 Da, auf. Spezielle nichtionische Siliconpolymere, welche das zuvor angeführte mittlere Molekulargewicht $M_w$ aufweisen, resultieren in einer besonders hohen Pflege keratinischer Fasern nach der Farbveränderung bei gleichzeitig minimaler und mit dem ungeschulten menschlichen Auge nicht sichtbaren Farbverschiebung von $\Delta E < 2$. Das mittlere Molekulargewicht $M_w$ kann beispielsweise durch Gelpermeationschromatographie (GPC) bestimmt werden (Liu X. M. et. al.; "Comparative Studies of Poly(DimethylSiloxanes) Using Automated GPC-MALDI-TOF MS and On-Line GPC-ESI-TOF MS"; Am. Soc. Mass. Spectrom., 2003, 14, Seiten 195 bis 202).

Der Einsatz der zuvor genannten Gesamtmenge des speziellen nichtionischen Siliconpolymers führt zu einer erhöhten Pflege der keratinischen Fasern, ohne jedoch das Färberesultat in Form einer sichtbaren Farbverschiebung zu beeinflussen.

Es hat sich gezeigt, dass ein Zusatz an Polyoxyethylen(20)-sorbitan monolaurat das mindestens eine nichtionische Siliconpolymer der Formel (III), in den erfindungsgemäßen kosmetischen Mitteln stabilisieren kann, so dass die Pflegeeffekte weiter verstärkt werden. Erfindungsgemäß bevorzugte kosmetische Mittel enthalten daher zusätzlich Polyoxyethylen(20)-sorbitan monolaurat in einer Gesamtmenge von 0,003 bis 1,5 Gew.-%, vorzugsweise von 0,006 bis 1,1 Gew.-%, bevorzugt von 0,009 bis 0,8 Gew.-%, weiter bevorzugt von 0,01 bis 0,5 Gew.-%, insbesondere von 0,015 bis 0,3 Gew.-%, bezogen auf das Gesamtgewicht des kosmetischen Mittels.

Das mindestens eine nichtionische Siliconpolymer der Formel (III), kann weiterhin durch den Zusatz von Polyoxyethylen(7)-laurylether stabilisiert werden. Es ist daher im Rahmen der vorliegenden Erfindung bevorzugt, wenn die erfindungsgemäßen kosmetischen Mittel zusätzlich Polyoxyethylen(7)-laurylether in einer Gesamtmenge von 0,01 bis 6 Gew.-%, vorzugsweise von 0,02 bis 4,5 Gew.-%, bevorzugt von 0,03 bis 3 Gew.-%, weiter bevorzugt von 0,04 bis 2,1 Gew.-%, insbesondere von 0,05 bis 1,5 Gew.-%, bezogen auf das Gesamtgewicht des kosmetischen Mittels, enthalten.

Die erfindungsgemäßen kosmetischen Mittel können weitere Wirk- und Zusatzstoff enthalten. Es ist daher im Rahmen der vorliegenden Erfindung bevorzugt, wenn das kosmetische Mittel zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

Bevorzugt werden die erfindungsgemäßen kosmetischen Mittel als fließfähige Zubereitungen formuliert. Dabei sollten die kosmetischen Mittel so formuliert werden, dass diese sich einerseits gut am Anwendungsort auftragen und verteilen lassen, andererseits jedoch ausreichend viskos sind, so dass sie während der Einwirkzeit am Wirkort verbleiben und nicht verlaufen.

Es hat sich daher erfindungsgemäß als vorteilhaft erwiesen, wenn die erfindungsgemäßen kosmetischen Mittel mindestens ein Verdickungsmittel aus der Gruppe von (i) anionischen, synthetischen Polymeren; (ii) kationischen, synthetischen Polymeren; (iii) natürlich vorkommenden Verdickungsmitteln, wie nichtionischen Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektinen, Alginaten, Stärke-Fraktionen und Derivaten, wie Amylose, Amylopektin und Dextrinen, sowie Cellulosederivaten, wie beispielsweise Methylcellulose, Carboxyalkylcellulosen und Hydroxyalkylcellulosen; (iv) nichtionischen, synthetischen Polymeren, wie Polyvinylalkohol oder Polyvinylpyrrolidinon; (v) anorganischen Verdickungsmitteln, insbesondere Schichtsilikaten wie beispielsweise Bentonit, besonders Smektite, wie Montmorillonit oder Hectorit; sowie (vi) deren Mischungen, in einer Gesamtmenge von 0,0005 bis 5,0 Gew.-%, vorzugsweise von 0,001 bis 3,0 Gew.-%, bevorzugt von 0,005 bis 1,0 Gew.-%, insbesondere von 0,008 bis 0,01 Gew.-%, bezogen auf das Gesamtgewicht des kosmetischen Mittels, enthalten.

Es hat sich im Rahmen der vorliegenden Erfindung als vorteilhaft erwiesen, wenn als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,1 Gew.-%, bezogen auf das Gesamtgewicht des kosmetischen Mittels, enthalten ist.

Im Rahmen der vorliegenden Erfindung kann es bevorzugt sein, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 1,0 bis 35 Gew.-%, vorzugsweise von 5,0 bis 30 Gew.-%, bevorzugt von 10 bis 25 Gew.-%, insbesondere von 12 bis 20 Gew.-%, bezogen auf das Gesamtgewicht des kosmetischen Mittels, enthalten ist.

Bevorzugt können die erfindungsgemäßen kosmetischen Mittel weiterhin mindestens einen Partialester aus einem Polyol mit 2 bis 6 Kohlenstoffatomen und linearen gesättigten Carbonsäuren mit 12 bis 30, insbesondere 14 bis 22 Kohlenstoffatomen, wobei die Partialester hydroxyliert sein können, in einer Gesamtmenge von 0,5 bis 10 Gew.-%, insbesondere von 3,0 bis 8,0 Gew.-%, bezogen auf das Gesamtgewicht des kosmetischen Mittels, enthalten. Solche Partialester sind insbesondere die Mono- und Diester von Glycerin oder die Monoester von Propylenglycol oder die Mono- und Diester von Ethylenglycol oder die Mono-, Di-, Tri- und Tetraester von Pentaerythrit jeweils mit linearen gesättigten $C_{12}$ - $C_{30}$-Carbonsäuren, welche hydroxyliert sein können, insbesondere diejenigen mit Palmitin- und Stearinsäure, die Sorbitanmono-, -di- oder -triester von linearen gesättigten $C_{12}$ -$C_{30}$-Carbonsäuren, welche hydroxyliert sein können, insbesondere diejenigen von Myristinsäure, Palmitinsäure, Stearinsäure oder von Mischungen dieser Fettsäuren und die Methylglucosemono- und -diester von linearen gesättigten $C_{12}$ - $C_{30}$-Carbonsäuren, welche hydroxyliert sein können.

Im Rahmen der vorliegenden Erfindung kann es vorgesehen sein, dass die erfindungsgemäßen kosmetischen Mittel mindestens einen Polyolpartialester, ausgewählt aus Glycerinmonostearat, Glycerinmonopalmitat, Glycerindistearat, Glycerindipalmitat, Ethylenglycolmonostearat, Ethylenglycolmonopalmitat, Ethylenglycoldistearat, Ethylenglycoldipalmitat, sowie Mischungen hiervon, insbesondere Mischungen aus Glycerinmonostearat, Glycerinmonopalmitat, Glycerindistearat und Glycerindipalmitat in einer Gesamtmenge von 0,5 bis 10 Gew.-%, insbesondere von 3,0 bis 8,0 Gew.-%, bezogen auf das Gesamtgewicht des kosmetischen Mittels, enthalten.

Der Einsatz der zuvor angeführten Alkohole, Partialester und Polypartialester in den erfindungsgemäßen kosmetischen Mitteln kann insbesondere dann bevorzugt sein, wenn die erfindungsgemäßen kosmetischen Mittel in Form einer cremeförmigen Öl-in-Wasser-Emulsion vorliegen.

Weiterhin kann es erfindungsgemäß vorgesehen sein, dass die kosmetischen Mittel gemäß der Erfindung mindestens ein Tensid enthalten. Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Gemäß einer bevorzugten Ausführungsform der vorliegenden Erfindung enthalten die erfindungsgemäßen kosmetischen Mittel mindestens ein amphoteres Tensid in einer Gesamtmenge von 0,1 bis 5,0 Gew.-%, insbesondere von 0,2 bis 2,0 Gew.-%, bezogen auf das Gesamtgewicht des kosmetischen Mittels. Als amphotere bzw. zwitterionische Tenside werden solche oberflächenaktiven Verbindungen bezeichnet, welche mindestens eine quartäre Ammoniumgruppe und mindestens eine $-COO^{(-)}$-oder-$SO3^{(-)}$-Gruppe aufweisen.

Als amphotere Tenside sind im Rahmen der vorliegenden Erfindung die nachfolgend genannten Verbindungen besonders bevorzugt:

- Alkylbetaine mit 8 bis 20 Kohlenstoffatomen in der Alkylgruppe,
- Amidopropylbetaine mit 8 bis 20 Kohlenstoffatomen in der Acylgruppe,
- Sulfobetaine mit 8 bis 20 Kohlenstoffatomen in der Acylgruppe, und
- Amphoacetate oder Amphodiacetate mit 8 bis 20 Kohlenstoffatomen in der Acylgruppe.

In einer besonders bevorzugten Ausführungsform enthalten die erfindungsgemäßen kosmetischen Mittel als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das Gesamtgewicht des kosmetischen Mittels.

Weiterhin kann es vorgesehen sein, dass die erfindungsgemäßen kosmetischen Mittel mindestens ein ethoxyliertes nichtionisches Tensid in einer Gesamtmenge von 0,5 bis 6,0 Gew.-%, insbesondere von 1,0 bis 4,0 Gew.-%, bezogen auf das Gesamtgewicht des kosmetischen Mittels, enthalten. Dabei hat es sich als besonders vorteilhaft erwiesen, wenn das ethoxylierte nichtionische Tensid einen HLB-Wert oberhalb von 10, vorzugsweise oberhalb von 13 aufweist. Hierzu ist es erforderlich, dass das nichtionische Tensid einen ausreichend hohen Ethoxylierungsgrad besitzt. In diesem Zusammenhang enthält das erfindungsgemäße kosmetische Mittel daher als ethoxyliertes nichtionisches Tensid mindestens ein ethoxyliertes Tensid mit mindestens 12 Ethylenoxideinheiten. Neben den entsprechend ethoxylierten Fettalkoholen, insbesondere Laurylalkohol, Myristylalkohol, Cetylalkohol, Stearylalkohol, Arachylalkohol und Behenylalkohol, sind erfindungsgemäß insbesondere die Anlagerungsprodukte von 20 bis 60 Mol Ethylenoxid an Rizinusöl und gehärtetes Rizinusöl besonders geeignet. Das mindestens eine ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30.

Kosmetische Mittel im Rahmen der vorliegenden Erfindung weisen in der Regel einen basischen pH-Wert, insbesondere zwischen pH 8,0 und pH 12, auf. Diese pH-Werte sind erforderlich, um eine Öffnung der äußeren Schuppenschicht (Cuticula) zu gewährleisten und eine Penetration der Oxidationsfarbstoffvorprodukte und/oder des Oxidationsmittels in das Haar zu ermöglichen.

Die Einstellung des zuvor genannten pH-Wertes kann bevorzugt unter Verwendung eines Alkalisierungsmittels erfolgen. Im Rahmen der vorliegenden Erfindung ist das Alkalisierungsmittel ausgewählt aus der Gruppe von (i) anorganischen Alkalisierungsmitteln; (ii) organischen Alkalisierungsmitteln; sowie (iii) deren Mischungen, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels, enthalten.

Bevorzugte anorganische Alkalisierungsmittel sind ausgewählt aus der Gruppe, welche gebildet wird aus Ammoniak bzw. Ammoniumhydroxid, also wässrigen Lösungen von Ammoniak, Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Ammoniak bzw. Ammoniumhydroxid ist ein besonders bevorzugtes Alkalisierungsmittel. Besonders bevorzugt ist Ammoniak in einer Gesamtmenge von 0,1 bis 20 Gew.-%, vorzugsweise von 0,5 bis 10 Gew.-%, insbesondere von 1,0 bis 7,0 Gew.-%, bezogen auf das Gesamtgewicht des kosmetischen Mittels, enthalten.

Bevorzugte organische Alkalisierungsmittel sind ausgewählt aus mindestens einem Alkanolamin. Erfindungsgemäß bevorzugte Alkanolamine sind ausgewählt aus Alkanolaminen aus primären, sekundären oder tertiären Aminen mit einem $C_2$-$C_6$-Alkylgrundkörper, welcher mindestens eine Hydroxylgruppe trägt. Besonders bevorzugte Alkanolamine werden aus der Gruppe ausgewählt, welche gebildet wird aus 2-Aminoethan-1-ol (Monoethanolamin), 3-Aminopropan-1-ol, 4-Amino-butan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine werden ausgewählt aus der Gruppe von 2-Aminoethan-1-ol (Monoethanolamin), 2-Amino-2-methylpropan-1-ol, 2-Amino-2-methyl-propan-1,3-diol und Triethanolamin. Besonders bevorzugte erfindungsgemäße kosmetische Mittel enthalten eine Mischung aus Monoethanolamin und 2-Amino-2-methylpropan-1ol. Bevorzugt ist das mindestens eine Alkanolamin in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels, enthalten.

Weitere erfindungsgemäß bevorzugte organische Alkalisierungsmittel sind ausgewählt aus basischen Aminosäuren, besonders bevorzugt ausgewählt aus der Gruppe, welche gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte erfindungsgemäße kosmetische Mittel enthalten mindestens ein von Alkanolaminen und Ammoniak verschiedenes Alkalisierungsmittel in einer Gesamtmenge von 0,05 bis 5,0 Gew.-%, insbesondere von 0,5 bis 3,0 Gew.-%, bezogen auf das Gesamtgewicht des kosmetischen Mittels.

In einer besonders bevorzugten Ausführungsform enthalten die erfindungsgemäßen kosmetischen Mittel als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels.

Bevorzugt beträgt der pH-Wert der erfindungsgemäßen kosmetischen Mittel, gemessen bei 22°C, 8 bis 13, vorzugsweise 9,5 bis 12, bevorzugt 10 bis 11,5, insbesondere 10,5 bis 11.

Im Rahmen der vorliegenden Erfindung kann es weiterhin bevorzugt sein, wenn die erfindungsgemäßen kosmetischen Mittel mindestens ein Öl, ausgewählt aus der Gruppe von Sonnenblumenöl, Maisöl, Sojaöl, Kürbiskernöl, Traubenkernöl, Sesamöl, Haselnussöl, Aprikosenkernöl, Macadamianussöl, Araraöl, Rizinusöl, Avocadoöl sowie deren Mischungen, in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels, enthalten. Durch den Einsatz eines zuvor genannten Öls kann der Pflegeeffekt der nichtionischen Siliconpolymere weitergehend gesteigert werden.

Besonders bevorzugt enthalten die erfindungsgemäßen kosmetischen Mittel Traubenkernöl in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels.

Gemäß einer besonders bevorzugten Ausführungsform der vorliegenden Erfindung enthalten die als Öl-in-Wasser-Emulsion vorliegenden erfindungsgemäßen kosmetischen Mittel - bezogen auf das Gesamtgewicht der kosmetischen Mittel -

- Cetearylalkohol in einer Gesamtmenge von 2,0 bis 20 Gew.-%, insbesondere von 5,0 bis 12 Gew.-%, weiterhin
- Mischungen aus Glycerinmonostearat, Glycerinmonopalmitat, Glycerindistearat und Glycerindipalmitat in einer Gesamtmenge von 0,5 bis 10 Gew.-%, bevorzugt 3,0 bis 8,0 Gew.-%, weiterhin
- mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, weiterhin
- eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, weiterhin
- Traubenkernöl in einer Gesamtmenge von 0,1 bis 10 Gew.-%, bevorzugt von 0,2 bis 5,0 Gew.-%, insbesondere von 0,5 bis 2,0 Gew.-%.

Oxidative Färbezusammensetzungen können auch direkt vor der Anwendung aus zwei oder mehreren getrennt verpackten Zusammensetzungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme ist insbesondere dort bevorzugt, wo Inkompatibilitäten der Inhaltsstoffe zu erwarten oder zu befürchten sind. Die oxidative Färbezusammensetzung wird in diesen Fällen vom Verbraucher direkt vor der Anwendung durch Vermischen der Komponenten hergestellt. Im Rahmen der vorliegenden Erfindung ist dieses Vorgehen bei oxidativen Färbemitteln, bei welchen das erfindungsgemäße kosmetische Mittel zunächst getrennt von einer Oxidationsmittelzubereitung, enthaltend mindestens ein Oxidationsmittel, vorliegt, besonders bevorzugt.

Ein weiterer Gegenstand der vorliegenden Erfindung ist daher eine Verpackungseinheit (Kit-of-Parts), umfassend - getrennt voneinander konfektioniert -

a) mindestens einen Container (C1), enthaltend ein erfindungsgemäßes kosmetisches Mittel, und
b) mindestens einen Container (C2), enthaltend eine Oxidationsmittelzubereitung, welche in einem kosmetisch verträglichen Träger mindestens ein Oxidationsmittel in einer Gesamtmenge von 0,5 bis 7,0 Gew.-%, vorzugsweise von 1,0 bis 7,0 Gew.-%, insbesondere von 3,0 bis 7,0 Gew.-%, bezogen auf das Gesamtgewicht der Oxidationsmittelzubereitung, sowie mindestens eine Säure enthält.

Der Einsatz des mindestens einen nichtionischen Siliconpolymers in Kombination mit bestimmten Mengen an Oxidationsmittel resultiert bei Verwendung der zuvor genannten Verpackungseinheit zur Färbung keratinischer Fasern überraschenderweise in einer erhöhten Pflege, insbesondere in einer erhöhten Nasskämmbarkeit, ohne dass jedoch durch den Zusatz des nichtionischen Siliconpolymers eine mit dem ungeschulten menschlichen Auge wahrnehmbare Farbverschiebung von $\Delta E > 2$ auftritt.

Unter dem Begriff "Container" wird im Rahmen der vorliegenden Erfindung eine Umhüllung verstanden, welche in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich dabei jedoch um Umhüllungen aus Glas oder Kunststoff.

Die Oxidationsmittel im Rahmen der vorliegenden Erfindung sind von Luftsauerstoff verschieden. Als Oxidationsmittel können Wasserstoffperoxid sowie dessen feste Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Erfindungsgemäß bevorzugt ist das Oxidationsmittel daher ausgewählt aus der Gruppe von Persulfaten, Chloriten, Wasserstoffperoxid und Anlagerungsprodukten von Wasserstoffperoxid an Harnstoff, Melamin sowie Natriumborat, insbesondere Wasserstoffperoxid.

Ein besonders bevorzugte Ausführungsform der vorliegenden Erfindung ist somit dadurch gekennzeichnet, dass als Oxidationsmittel Wasserstoffperoxid in einer Gesamtmenge von 0,5 bis 7,0 Gew.-%, vorzugsweise von 1,0 bis 7,0 Gew.-%, insbesondere von 3,0 bis 7,0 Gew.-%, bezogen auf das Gesamtgewicht der Oxidationsmittelzubereitung, enthalten ist. Die Berechnung der Gesamtmenge bezieht sich hierbei auf 100 %-iges $H_2O_2$.

Die Oxidationsmittelzubereitungen können weiterhin Wasser in einer Gesamtmenge von 40 bis 98 Gew.-%, insbesondere von 65 bis 85 Gew.-%, bezogen auf das Gesamtgewicht der Oxidationsmittelzubereitung, enthalten.

Gemäß einer bevorzugten Ausführungsform der vorliegenden Erfindung enthalten die Oxidationsmittelzubereitungen weiterhin mindestens ein lineares gesättigtes Alkanol mit 12 bis 30 Kohlenstoffatomen, insbesondere mit 16 bis 22 Kohlenstoffatomen, in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung. Bevorzugt sind insbesondere Cetylalkohol, Stearylalkohol, Arachidylalkohol, Behenylalkohol und Lanolinalkohol oder Gemische dieser Alkohole, wie sie bei der großtechnischen Hydrierung von pflanzlichen und tierischen Fettsäuren erhältlich sind, sowie Mischungen dieser Alkanole. Besonders bevorzugt ist die Mischung Cetearylalkohol.

In einer weiteren bevorzugten Ausführungsform der vorliegenden Erfindung enthalten die Oxidationsmittelzubereitungen mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1 bis 4,0 Gew.-%, bezogen auf das Gesamtgewicht der Oxidationsmittelzubereitung.

Im Rahmen der vorliegenden Erfindung kann es darüber hinaus auch vorgesehen sein, dass die Oxidationsmittelzubereitungen mindestens einen Ester aus einer Carbonsäure mit 10 bis 20 Kohlenstoffatomen und einem linearen oder verzweigten Alkohol mit 1 bis 5 Kohlenstoffatomen, insbesondere Isopropylmyristat, in einer Gesamtmenge von 3,0 bis 25 Gew.-%, vorzugsweise von 5,0 bis 20 Gew.-%, insbesondere von 8,0 bis 15 Gew.-%, bezogen auf das Gesamtgewicht der Oxidationsmittelzubereitung, enthalten.

Gemäß einer besonders bevorzugten Ausführungsform der vorliegenden Erfindung enthalten die Oxidationsmittelzubereitungen - bezogen auf das Gesamtgewicht der Oxidationsmittelzubereitungen -

- mindestens ein lineares gesättigtes Alkanol mit 12 bis 30 Kohlenstoffatomen in einer Gesamtmenge von 0,1 bis 10 Gew.-%, vorzugsweise von 0,5 bis 5,0 Gew.-%, insbesondere von 1,0 bis 4,0 Gew.-%, weiterhin
- mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1,0 bis 4,0 Gew.-%, sowie
- mindestens einen Ester aus einer Carbonsäure mit 10 bis 20 Kohlenstoffatomen und einem linearen oder verzweigten Alkohol mit 1 bis 5 Kohlenstoffatomen, bevorzugt Isopropylmyristat, in einer Gesamtmenge von 3,0 bis 25 Gew.-%, vorzugsweise von 5,0 bis 20 Gew.-%, insbesondere von 8,0 bis 15 Gew.-%.

Die erfindungsgemäßen Oxidationsmittelzubereitungen enthalten weiterhin mindestens eine Säure. Bevorzugte Säuren sind ausgewählt aus Dipicolinsäure, Genusssäuren, wie beispielsweise Zitronensäure, Essigsäure, Apfelsäure, Milchsäure und Weinsäure, verdünnten Mineralsäuren, wie Salzsäure, Phosphorsäure, Pyrophosphorsäure und Schwefelsäure, sowie Mischungen hiervon. Die Oxidationsmittelzubereitungen weisen bevorzugt einen pH-Wert im Bereich von 2 bis 5, insbesondere von 3 bis 4, auf.

Zur Herstellung von oxidativen Färbezusammensetzungen aus der erfindungsgemäßen Verpackungseinheit (Kit-of-parts) wird das erfindungsgemäße kosmetische Mittel in dem Container C1 mit der Oxidationsmittelzubereitung in dem Container C2 oder *vice versa* vermischt.

Weiterhin kann es erfindungsgemäß besonders vorteilhaft sein, wenn die Verpackungseinheit mindestens ein weiteres Haarbehandlungsmittel in einem zusätzlichen Container enthält, insbesondere eine Konditioniermittelzubereitung. Diese Konditioniermittelzubereitung enthält vorteilhafterweise mindestens ein Konditioniermittel, ausgewählt aus der Gruppe von kationischen Polymeren, Siliconderivaten und Ölen. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, welche das Abteilen von Faserbündeln bzw. Strähnchen aus der Gesamtmenge der Fasern erlaubt und vereinfacht.

Bezüglich des erfindungsgemäßen kosmetischen Mittels in dem Container C1 und der Oxidationsmittelzubereitung in dem Container C2 gilt mutatis mutandis das zu den erfindungsgemäßen kosmetischen Mitteln Gesagte.

Bezüglich weiterer bevorzugter Ausführungsformen der erfindungsgemäßen Verwendung gilt mutatis mutandis das zu den erfindungsgemäßen kosmetischen Mitteln sowie zu der erfindungsgemäßen Verpackungseinheit Gesagte.

Die nachfolgenden Beispiele sollen bevorzugte Ausführungsformen der Erfindung erläutern, ohne sie jedoch einzuschränken.

Beispiele:

1. Rezepturen

Zusammensetzungen der eingesetzten kosmetischen Mittel (Öl-in-Wasser-Emulsionen, alle Mengen in Gew.-%). Das in den nachfolgenden Formulierungen verwendete nichtionische Siliconpolymer der Formel (III) mit n = 2 oder 3, m = 5 bis 9 und einem mittleren Molekulargewicht $M_w$ von 3.000 bis 100.000 Da.

| Rohstoff | V1 | E1* | E2* |
|---|---|---|---|
| Xanthan Gum | 0,05 | 0,05 | 0,05 |
| 2-Octyldodecanol | 2,3 | 2,3 | 2,3 |
| Lanette N [a)] | 14 | 14 | 14 |
| Cetearyl Alkohol | 3,9 | 3,9 | 3,9 |
| Glycerinmonostearat | 6,0 | 6,0 | 6,0 |
| Glycerol 99,5 % | 2,0 | 2,0 | 2,0 |
| Kokosamidopropylbeatin, 40%ig | 2,0 | 2,0 | 2,0 |
| Monoethanolamin | 4,5 | 4,5 | 4,5 |
| 2-Amino-2-methylpropanol | 0,10 | 0,10 | 0,10 |
| Natriumsulfit, wasserfrei | 0,15 | 0,15 | 0,15 |
| Caramelsirup, 75%ig | 0,10 | 0,10 | 0,10 |
| Traubenkernöl | 1,0 | 1,0 | 1,0 |
| p-Toluylendiaminsulfat | 0,032 | 0,032 | 0,032 |
| 4-Amino-3-methylphenol | 0,32 | 0,32 | 0,32 |
| Resorcinol | 0,037 | 0,037 | 0,037 |
| 1-Naphthol | 0,092 | 0,092 | 0,092 |
| p-Amino-o-cresol | 0,21 | 0,21 | 0,21 |
| 2-Amino-6-chloro-4-nitrophenol | 0,24 | 0,24 | 0,24 |

(fortgesetzt)

| Rohstoff | V1 | E1* | E2* |
|---|---|---|---|
| Nichtionisches Siliconpolymer ** | - | 0,25 | 0,5 |
| Wasser, vollentsalzt | ad 100,00 | ad 100,00 | ad 100,00 |
| * erfindungsgemäß<br>** Aktivsubstanz<br>a) INCI-Bezeichnung: Cetearyl alcohol, Sodium cetearyl sulfate (BASF) | | | |

Die Fettbasis wurde jeweils zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V1 handelt es sich um eine nicht erfindungsgemäße Vergleichsrezeptur ohne nichionisches Siliconpolymer. Die Rezepturen E1 und E2 sind erfindungsgemäße Beispiele.

Oxidationsmittelzubereitung O1 (alle Mengen in Gew.-%)

| Rohstoff | O1 |
|---|---|
| Dinatriumpyrophosphat | 0,10 |
| Dipicolinsäure | 0,10 |
| Kaliumhydroxid 50% | 0,22 |
| 1-Hydroxyethan-1,1-diphosphonsäure 60% | 0,25 |
| Emulgade F b) | 4,0 |
| Cetearyl Alcohol | 0,5 |
| Ceteareth-20 | 0,5 |
| Bienenwachs | 0,3 |
| Isopropylmyristat | 10 |
| Wasserstoffperoxid 50 % | 11 |
| Wasser vollentsalzt | ad 100 |
| b) INCI-Bezeichnung: Cetearyl alcohol, PEG-40 Castor oil, Sodium cetearyl sulfat (BASF) | |

2. Geringe Farbverschiebung durch den Zusatz des nichtionischen Siliconpolymers

Zur Herstellung der oxidativen Färbemittel für die Bestimmung der Farbverschiebung wurden die kosmetischen Mittel V1 sowie E1 und E2 jeweils im Gewichtsverhältnis 1:1 mit der obigen Oxidationsmittelzubereitung O1 vermischt.

Die auf diese Weise hergestellten oxidativen Färbemittel wurden jeweils in definierter Menge (4 g oxidatives Färbemittel pro 1 g Yakhaar) auf Yakhaar-Strähnen aufgetragen (jeweils 12 Strähnen pro oxidativem Färbemittel) und verblieben für eine Einwirkdauer von 30 Minuten bei 32 °C auf den Haarsträhnen. Anschließend wurden die verbliebenen Mittel jeweils 2 Minuten lang mit lauwarmem Wasser aus den Haarsträhnen ausgespült, die Strähnen zunächst mit einem Handtuch getrocknet und anschließend trocken geföhnt.

Alle Strähnen wurden mit einem Farbmessgerät der Firma Datacolor, Typ Spectraflash 450, vermessen. Die für die Beurteilung Farbverschiebung herangezogenen Werte ΔE ergeben sich aus den an der jeweiligen Strähne gemessenen L*a*b-Farbmesswerten wie folgt:

$$\Delta E = ((L_i-L_0)^2 + (a_i-a_0)^2 + (b_i-b_0)^2)^{1/2}$$

$a_0$, $b_0$ und $L_0$ sind hierbei jeweils die Mittelwerte der aus den 12 Messungen ermittelten Farbmesswerte der unter Verwendung des standardisierten Trägers ausgefärbten Yakhaar-Strähnen. $a_i$, $b_i$ und $L_i$ stehen jeweils für die Mittelwerte der Farbmesswerte, welche für ausgefärbte Yakhaar-Strähnen unter Zusatz des speziellen nichtionischen Siliconpolymers zu dem standardisierten Träger erhalten werden.

Je größer der Wert für ΔE ist, desto stärker ausgeprägt ist die Farbdifferenz oder "Farbverschiebung". Farbdifferenzen mit ΔE < 1 sind für das menschliche Auge nicht wahrnehmbar. Farbdifferenzen mit ΔE < 2 sind für das geschulte Auge sichtbar. Farbdifferenzen mit ΔE > 2 sind auch für das ungeschulte Auge sichtbar. In Tabelle 1 sind die ΔE-Werte für die Ausfärbungen unter Verwendung der kosmetischen Mittel E1 und E2 dargestellt. Die Ausfärbungen mit den erfindungsgemäßen kosmetischen Mitteln E1 und E2, welche mindestens ein spezielles nichtionisches Silicon in einer Gesamtmenge von 0,25 Gew.-% bzw. 0,5 Gew.-% enthalten, weisen nur eine geringe Farbverschiebung von ΔE < 2 auf, welche für das ungeschulte Auge nicht sichtbar sind. Die Farbverschiebung, welche durch das nichtionische Siliconpolymer hervorgerufen wird, ist hierbei umso geringer, je kleiner die eingesetzte Menge des nichtionischen Siliconpolymers ist.

| Oxidatives Färbemittel | ΔE |
|---|---|
| E1 + O1 (1:1) | 1,085 |
| E2 + O1 (1:1) | 1,305 |

3. Verbesserte Pflege

Zur Herstellung der oxidativen Färbemittel zur Bestimmung der Pflege wurden die kosmetischen Mittel V1 und E2 jeweils im Gewichtsverhältnis 1:1 mit der obigen Oxidationsmittelzubereitung O1 vermischt.

12 Strähnen von natürlich hellbraunem europäischen Haar (IHIP (New York), lot # 03/2012, N104, lenght 15 cm, weight 1 g) wurden mit einer wässrigen Natrium-Laurylethersulfat-Lösung (3 % Aktivsubstanzgehalt in der Lösung) gewaschen. Die Strähnen wurden an der Luft getrocknet und für 24 h bei 25°C und 25% relativer Luftfeuchtigkeit gelagert. Nach Einweichen dieser Strähnen für 5 Minuten in Wasser wurde deren Nasskämmbarkeit bestimmt (Referenzwert).

Für die Färbungen wurden jeweils 12 Strähnen von natürlich europäischen Haar (IHIP (New York), lot # 03/2012, N104, lenght 15 cm, weight 1 g) je oxidativem Färbemittel verwendet. Hierfür wurden jeweils 4 g der unter Punkt 2. hergestellten oxidativen Färbemittel pro 1 g Haarsträhne appliziert. Nachdem die Strähnen für 30 min bei 32 °C gefärbt wurden, wurden sie für 2 min mit Wasser gespült und an der Luft getrocknet.

Die Messung der Nasskämmbarkeit wurde wie folgt durchgeführt:

Vor der Messung wurde jede Strähne unter Kämmen mit einem harten Gummikamm mit feinen Zähnen (Firma Hercules Sägemann, Hamburg Germany) für 2 Sekunden mit Wasser befeuchtet. Nachdem 3 Kämmvorgänge durchgeführt wurden, wird die Kämmkraft während weiteren 10 Kämmvorgängen gemessen, wobei die jeweilige Haarsträhne während des Kämmvorgangs langsam rotiert. Die erhaltenen Messwerte werden unter Verwendung der folgenden in der Software Statistica 10.0 (StatSoft Inc., USA) eingebetteten statistischen Tests verglichen:

- Shapiro-Wilks Test (Test für Normalverteilung)
- Ausreißertest nach Grubbs
- Bartlett-Test (Test auf Homoskedastizität von Varianzen)
- Univarianter Signifikanztest
- Newman-Keuls Test (Bestimmung von signifikanten Unterschieden)
- Unequal N HSD Test (Test auf Mehrfachvergleiche).

Die Änderung der Kämmkraft dK in Prozent kann mit Hilfe der Formel $dK = [(K_0-K_i)/K_0]*100$ berechnet werden. $K_0$ ist hierbei der Mittelwert der Kämmkraft für die ungefärbten Haarsträhnen und $K_i$ der Mittelwert für die mit dem jeweiligen oxidativen Färbemittel behandelten Haarsträhnen.

Die Pflege der Haarsträhnen ist umso höher, je geringer die aufgewendete Kämmkraft und damit je höher die Änderung der Kämmkraft ist. In Tabelle 2 sind die dK-Werte für die Ausfärbungen unter Verwendung der kosmetischen Mittel V1 und E2 dargestellt. Die Ausfärbung mit dem erfindungsgemäßen kosmetischen Mittel E2, welches mindestens ein spezielles nichtionisches Siliconpolymer in einer Gesamtmenge von 0,5 Gew.-% enthält, weist im Vergleich zu einer Ausfärbung ohne nichtionisches Siliconpolymer (V1) eine höhere Änderung der Kämmkraft und somit eine erhöhte Pflege auf.

| Oxidatives Färbemittel | dK [%] |
|---|---|
| V1 + O1 (1:1) | 36 |
| E2 + O1 (1:1) | 39 |

**Patentansprüche**

1. Kosmetisches Mittel zur Farbveränderung keratinischer Fasern, umfassend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,
b) mindestens ein nichtionisches Siliconpolymer in einer Gesamtmenge von 0,005 bis 0,5 Gew.-%, bezogen auf das Gesamtgewicht des kosmetischen Mittels, enthaltend mindestens eine Struktureinheit der Formel (I) und mindestens eine Struktureinheit der Formel (II)

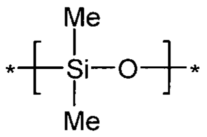
(I)
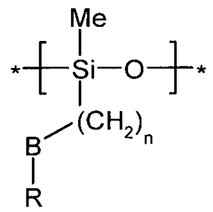
(II),

worin

n für ganze Zahlen von 1 bis 5 steht,
B für eine direkte Bindung oder ein Heteroatom aus der Gruppe von O, N oder S steht, und
R für eine Gruppe *-$(CH_2CH_2O)_x$-H oder *-$(CH_2CHMeO)_y$-H oder *-$(CH_2CH_2O)_x$-$(CH_2CHMeO)_y$-H steht, worin x und y, jeweils unabhängig voneinander, für ganze Zahlen von 2 bis 20 stehen,

wobei das kosmetische Mittel mindestens ein nichtionisches Siliconpolymer der Formel (III) enthält

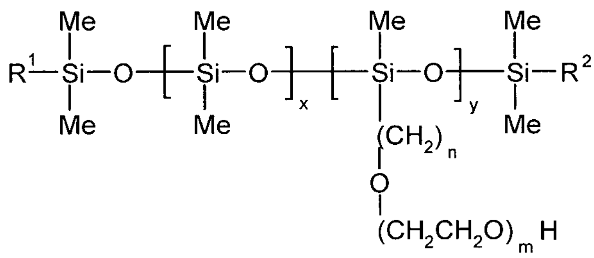
(III),

worin

$R^1$ und $R^2$, jeweils unabhängig voneinander, für eine Methylgruppe oder eine Hydroxylgruppe stehen,
x und y, jeweils unabhängig voneinander, für ganze Zahlen von 1 bis 1.000 stehen,
n für die ganzen Zahlen 2 oder 3 steht, und
m für ganze Zahlen von 5 bis 9 steht.

2. Kosmetisches Mittel nach Anspruch 1, **dadurch gekennzeichnet, dass** das mindestens eine nichtionische Siliconpolymer b) ein mittleres Molekulargewicht $M_w$ von 350 bis 200.000 Da, vorzugsweise von 500 bis 180.000 Da, bevorzugt von 1.000 bis 150.000 Da, insbesondere von 3.000 bis 100.000 Da, aufweist.

3. Kosmetisches Mittel nach einem der vorangehenden Ansprüche, **dadurch gekennzeichnet, dass** das kosmetische Mittel zusätzlich Polyoxyethylen(20)-sorbitan monolaurat in einer Gesamtmenge von 0,003 bis 1,5 Gew.-%, vorzugsweise von 0,006 bis 1,1 Gew.-%, bevorzugt von 0,009 bis 0,8 Gew.-%, weiter bevorzugt von 0,01 bis 0,5 Gew.-%, insbesondere von 0,015 bis 0,3 Gew.-%, bezogen auf das Gesamtgewicht des kosmetischen Mittels, enthält.

4. Kosmetisches Mittel nach einem der vorangehenden Ansprüche, **dadurch gekennzeichnet, dass** das kosmetische Mittel zusätzlich Polyoxyethylen(7)-laurylether in einer Gesamtmenge von 0,01 bis 6 Gew.-%, vorzugsweise von 0,02 bis 4,5 Gew.-%, bevorzugt von 0,03 bis 3 Gew.-%, weiter bevorzugt von 0,04 bis 2,1 Gew.-%, insbesondere

von 0,05 bis 1,5 Gew.-%, bezogen auf das Gesamtgewicht des kosmetischen Mittels, enthält.

**5.** Kosmetisches Mittel nach einem der vorangehenden Ansprüche, **dadurch gekennzeichnet, dass** das kosmetische Mittel zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

**6.** Kosmetisches Mittel nach Anspruch 5, **dadurch gekennzeichnet, dass** als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,1 Gew.-%, bezogen auf das Gesamtgewicht des kosmetischen Mittels, enthalten ist.

**7.** Kosmetisches Mittel nach einem der Ansprüche 5 oder 6, **dadurch gekennzeichnet, dass** als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das Gesamtgewicht des kosmetischen Mittels, enthalten ist.

**8.** Kosmetisches Mittel nach einem der Ansprüche 5 bis 7, **dadurch gekennzeichnet, dass** als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels, enthalten ist.

**9.** Verpackungseinheit (Kit-of-Parts), umfassend - getrennt voneinander konfektioniert -

a) mindestens einen Container (C1), enthaltend ein kosmetisches Mittel nach einem der Ansprüche 1 bis 8, und
b) mindestens einen Container (C2), enthaltend eine Oxidationsmittelzubereitung, welche in einem kosmetisch verträglichen Träger mindestens ein Oxidationsmittel in einer Gesamtmenge von 0,5 bis 7,0 Gew.-%, vorzugsweise von 1,0 bis 7,0 Gew.-%, insbesondere von 3,0 bis 7,0 Gew.-%, bezogen auf das Gesamtgewicht der Oxidationsmittelzubereitung, sowie mindestens eine Säure enthält.

## Claims

**1.** A cosmetic agent for changing the color of keratin fibers, comprising, in a cosmetically acceptable carrier,

a) at least one compound selected from the group of oxidation dye precursors, substantive dyes and mixtures thereof,
b) at least one non-ionic silicone polymer in a total amount of 0.005 to 0.5 wt.%, based on the total weight of the cosmetic agent, containing at least one structural unit of formula (I) and at least one structural unit of formula (II)

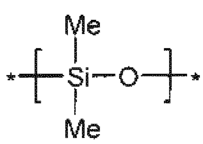
(I)

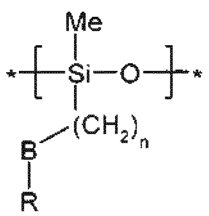
(II),

in which

n represents integers from 1 to 5,
B is a direct bond or a heteroatom from the group of O, N or S, and
R is a group $*\text{-}(CH_2CH_2O)_x\text{-}H$ or $*\text{-}(CH_2CHMeO)_y\text{-}H$ or $*\text{-}(CH_2CH_2O)_x\text{-}(CH_2CHMeO)_y\text{-}H$, where x and y, each independently of one another, are integers from 2 to 20,

wherein the cosmetic agent contains at least one non-ionic silicone polymer of formula (III)

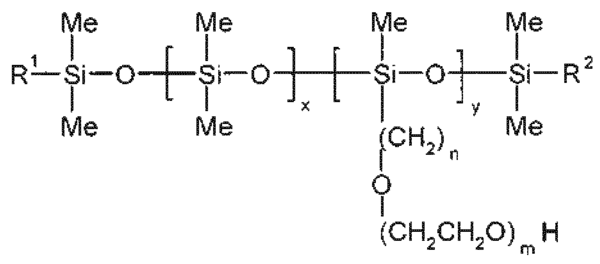

(III),

in which

$R^1$ and $R^2$ each represent, independently of one another, a methyl group or a hydroxyl group,
x and z each represent, independently of one another, an integer from 1 to 1,000,
n represents the integers 2 or 3, and
m represents integers from 5 to 9.

2. The cosmetic agent according to claim 1, **characterized in that** the at least one non-ionic silicone polymer b) has an average molecular weight $M_w$ of 350 to 200,000 Da, preferably 500 to 180,000 Da, more preferably 1,000 to 150,000 Da, in particular 3,000 to 100,000 Da.

3. The cosmetic agent according to one of the preceding claims, **characterized in that** the cosmetic agent additionally contains polyoxyethylene (20) sorbitan monolaurate in a total amount of 0.003 to 1.5 wt.%, preferably 0.006 to 1.1 wt.%, more preferably 0.009 to 0.8 wt.%, even more preferably 0.01 to 0.5 wt.%, in particular 0.015 to 0.3 wt.%, based on the total weight of the cosmetic agent.

4. The cosmetic agent according to one of the preceding claims, **characterized in that** the cosmetic agent additionally contains polyoxyethylene (7) lauryl ether in a total amount of 0.01 to 6 wt.%, preferably 0.02 to 4.5 wt.%, more preferably 0.03 to 3 wt.%, even more preferably 0.04 to 2.1 wt.%, in particular 0.05 to 1.5 wt.%, based on the total weight of the cosmetic agent.

5. The cosmetic agent according to one of the preceding claims, **characterized in that** the cosmetic agent additionally contains at least one further compound selected from the group of

(i) thickeners;
(ii) linear or branched, saturated or unsaturated alcohols having 8 to 20 carbon atoms;
(iii) surfactants, in particular amphoteric surfactants;
(iv) alkalizing agents;
(v) oils; and
(vi) mixtures thereof.

6. The cosmetic agent according to claim 5, **characterized in that** at least one naturally occurring thickener is contained as thickener, in particular xanthan gum and the salts thereof, in a total amount of 0.0005 to 5.0 wt.%, preferably 0.001 to 1.0 wt.%, more preferably 0.005 to 0.5 wt.%, in particular 0.01 to 0.1 wt.%, based on the total weight of the cosmetic agent.

7. The cosmetic agent according to one of claims 5 or 6, **characterized in that** at least one amphoteric surfactant selected from amidopropyl betaines having 9 to 13 carbon atoms in the acyl group is contained as surfactant in a total amount of 0.1 to 5.0 wt.%, in particular 0.2 to 2.0 wt.%, based on the total weight of the cosmetic agent.

8. The cosmetic agent according to one of claims 5 to 7, **characterized in that** a mixture of at least two mutually different alkanolamines, in particular of monoethanolamine and 2-amino-2-methylpropane-1-ol, is contained as alkalizing agent in a total amount of 0.05 to 15 wt.%, preferably 0.5 to 10 wt.%, in particular 3.5 to 7.5 wt.%, based on the total weight of the cosmetic agent.

9. Packaging unit (kit-of-parts), comprising - assembled separately -

a) at least one container (C1) containing a cosmetic agent according to one of claims 1 to 8, and
b) at least one container (C2) containing an oxidizing agent preparation which contains at least one oxidizing agent in a cosmetically acceptable carrier in a total amount of 0.5 to 7.0 wt.%, preferably 1.0 to 7.0 wt.%, in particular 3.0 to 7.0 wt.%, based on the total weight of the oxidizing agent preparation, and at least one acid.

## Revendications

1. Agent cosmétique destiné à modifier la couleur des fibres kératiniques, contenant, dans un support cosmétiquement acceptable,

a) au moins une liaison choisie dans le groupe des produits précurseurs de colorants par oxydation, des colorants montant directement sur la fibre ainsi que de leurs mélanges,
b) au moins un polymère de silicone non ionique en une quantité totale comprise entre 0,005 et 0,5 % en poids par rapport au poids total de l'agent cosmétique, contenant au moins une unité structurelle de formule (I) et/ou au moins une unité structurelle de formule (II).

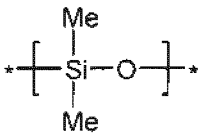 (I) 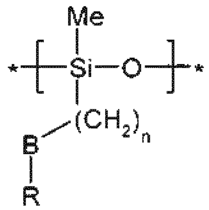 (II),

où

n représente des nombres entiers compris entre 1 et 5.
B représente une liaison directe ou un hétéroatome du groupe constitué de O, de N ou de S, et
R représente un groupe *-$(CH_2CH_2O)_x$-H ou *-$(CH_2CHMeO)_y$-H ou *-$(CH_2CH_2O)$x-$(CH_2CHMeO)_y$-H, x et y représentent chacun indépendamment des nombres entiers compris entre 2 et 20,

l'agent cosmétique contenant au moins un polymère de silicone non ionique de formule (III)

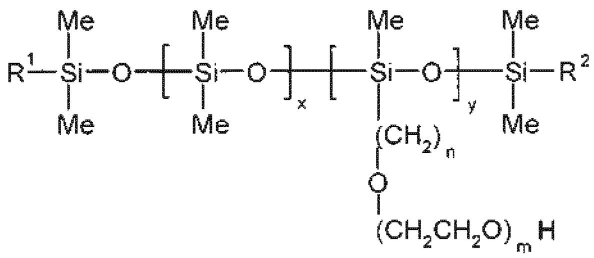 (III)

dans laquelle

$R^1$ et $R^2$ représentent chacun indépendamment un groupe méthyle ou un groupe hydroxyle,
x et y représentent chacun indépendamment des nombres entiers compris entre 1 et 1 000,
n représente les nombres entiers 2 ou 3, et
n représente des nombres entiers compris entre 5 et 9.

2. Agent cosmétique selon la revendication 1, **caractérisé en ce que** l'au moins un polymère de silicone non ionique b) présente un poids moléculaire moyen $M_w$ compris entre 350 et 200 000 Da, de préférence entre 500 et 180 000 Da, de manière préférée entre 1 000 et 150 000 Da, en particulier entre 3 000 et 100 000 Da.

3. Agent cosmétique selon l'une des revendications précédentes, **caractérisé en ce que** l'agent cosmétique contient en outre du monolaurate de polyoxyéthylène(20)-sorbitane en une quantité totale comprise entre 0,003 et 1,5 %

en poids, de préférence entre 0,006 et 1,1 % en poids, de manière préférée entre 0,009 et 0,8 % en poids, de manière davantage préférée entre 0,01 et 0,5 % en poids, en particulier entre 0,015 et 0,3 % en poids, par rapport au poids total de l'agent cosmétique.

**4.** Agent cosmétique selon l'une des revendications précédentes, **caractérisé en ce que** l'agent cosmétique contient en outre du polyoxyéthylène(7)-lauryléther en une quantité totale comprise entre 0,01 et 6 % en poids, de préférence entre 0,02 et 4,5 % en poids, de manière préférée entre 0,03 et 3 % en poids, de manière davantage préférée entre 0,04 et 2,1 % en poids, en particulier entre 0,05 et 1,5 % en poids, par rapport au poids total de l'agent cosmétique.

**5.** Agent cosmétique selon l'une des revendications précédentes, **caractérisé en ce que** l'agent cosmétique contient en outre au moins un autre composé choisi dans le groupe constitué par

i) des agents épaississants ;
ii) des alcools linéaires ou ramifiés, saturés ou insaturés, ayant 8 à 20 atomes de carbone ;
iii) des tensioactifs, en particulier des tensioactifs amphotères ;
iv) des agents d'alcalinisation ;
v) des huiles ; et
vi) par leurs mélanges.

**6.** Agent cosmétique selon la revendication 5, **caractérisé en ce qu'**au moins un agent épaississant naturel, en particulier la gomme de xanthane ainsi que ses sels, est présent comme agent épaississant, en une quantité totale comprise entre 0,0005 et 5,0 % en poids, de préférence entre 0,001 et 1,0 % en poids, de manière préférée entre 0,005 et 0,5 % en poids, en particulier entre 0,01 à 0,1 % en poids, par rapport au poids total de l'agent cosmétique.

**7.** Agent cosmétique selon l'une quelconque des revendications 5 ou 6, **caractérisé en ce qu'**au moins un tensioactif amphotère choisi parmi les amidopropylbétaïnes ayant 9 à 13 atomes de carbone dans le groupe acyle est présent comme tensioactif, en une quantité totale comprise entre 0,1 et 5,0 % en poids, en particulier entre 0,2 et 2,0 % en poids, par rapport au poids total de l'agent cosmétique.

**8.** Agent cosmétique selon l'une des revendications 5 à 7, **caractérisé en ce qu'**un mélange d'au moins deux alcanolamines différentes l'une de l'autre, en particulier de monoéthanolamine et de 2-amino-2-méthylpropan-1-ol, est présent comme alcalinisant, en une quantité totale comprise entre 0,05 et 15 % en poids, de préférence entre 0,5 et 10 % en poids, en particulier entre 3,5 et 7,5 % en poids, par rapport au poids total de l'agent cosmétique.

**9.** Unité de conditionnement (Kits-of-Parts) comprenant, confectionnés séparément les uns des autres,

a) au moins un récipient (C1) contenant un agent cosmétique selon l'une des revendications 1 à 8, et
b) au moins un récipient (C2) contenant une préparation d'agent oxydant qui contient, dans un support cosmétiquement acceptable, au moins un agent oxydant en une quantité totale comprise entre 0,5 et 7,0 % en poids, de préférence entre 1,0 et 7,0 % en poids, en particulier entre 3,0 et 7,0 % en poids, par rapport au poids total de la préparation d'agent oxydant, ainsi qu'au moins un acide.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 547790 A1
- US 20020144356 A1
- EP 2559456 A1
- US 20100229314 A


### In der Beschreibung aufgeführte Nicht-Patentliteratur


- **LIU X. M.** Comparative Studies of Poly(DimethylSiloxanes) Using Automated GPC-MALDI-TOF MS and On-Line GPC-ESI-TOF MS. *Am. Soc. Mass. Spectrom.,* 2003, vol. 14, 195-202